# EUROPEAN PATENT APPLICATION

(11) **EP 3 881 790 A1**
(43) Date of publication of application: **22.09.2021**
(21) Application number: 21000076.6
(22) Date of filing: 15.03.2021
(51) Int. Cl.: A61B 18/26, A61N 5/06

(54) **USING PHOTOHYDRAULICAL MECHANIC SHOCK, FOR SELEKTIVE DESTRUCTION OF CELL MEMBRANES OF CANCER CELLS**

(30) Priority: 15.03.2020 SE 2000055
(71) Applicant: Denev, Martin Ivanov, 554 63 Jönköping (SE)
(72) Inventor: Denev, Martin Ivanov, 554 63 Jönköping (SE)

(57) **Abstract**

THE INVENTION BELONGS TO THE AREA OF MEDICAL TEKHNIK.

THE INVENTION IS USING OF A KNOWN TEKHNIK, THE PHOTOHYDRAULIKAL EFFEKT TECHNIK, FOR A NEW UNKNOWN USING, FOR SELEKTIV DESTRUKTION OF THE RELATIVELY WEAKER CELLSMEMBRAN OF THE CANCERCELLS, WHICH HAS RELATIVELI WEAKER CELLMEMBRAN THAN HEALTHY CELLS.

PHOTOHYDRAULIKAL EFFEKT IS AN EXPLOSION OF GAS, AS AIR, IN THE WATER, WHEN THE LASER BEAM IS TARGETING GAS, AS AIR, IN THE WATER.

BY REGULATION OF THE LASERS EFFEKT IS REGULATED THE EFFEKT OF THE MECHANIKAL CHOCK, **WHICH SELEKTIV IS DESTROING THE MEMBRANE OF THE CANCERCELLS, WHITOUT TO DESTROY THE MEMBRANE OF THE HEALTHY CELLS.**

**THE PHOTOHYDRAULIKAL MECHANIKAL CHOCK IS USED TOO AND FOR DESTRUKTION OF BACTERIA, VIRUSES, STONS AND SAND.**

## Description

THE INVENTION BELONGS TO THE AREA OF MEDICAL TEKHNIK.

THE INVENTION IS USING A KNOWN TEKHNIK, THE PHOTOHYDRAULIKAL EFFEKT TECHNIK, FOR NEW UNKNOWN USING, FOR SELEKTIV DESTRUKTION OF THE RELATIVELY WEAKER MEMBRAN OF THE CANCERCELLS, WITHOUT, IN THE SAME TIME, TO DESTROY THE MEMBRAN OF THE HEALTHY CELLS.

IT EXIST A TEKNIKAL PROBLEM TO PLAN THE LEVEL OF EFFEKT OF THE MEKHANIKAL CHOCK, WHICH MEKHANIKAL CHOCK VILL DESTROY THE MEMBRAN OF THE CANCERCELLS, WITHOUT TO DESTROY THE HEALTHY CELLS.

THIS TEKHNIKAL PROBLEM IS SOLVED BY REGULATION OF THE EFFEKT OF THE LASER, WHICH CAUSED THE MECHANIKAL CHOCK.

PHOTOHYDRAULIKAL EFFEKT IS MEKHANIKAL CHOCK, CAUSED BY THE EXPLOSION OF THE GAS, AS AIR, IN THE WATER, WHEN THE LASER BEAM IS TARGETING THE GAS, AS AIR, IN THE WATER.

## Claims

1. USING OF PHOTOHYDRAULIKAL MEKHANIK CHOCK FOR SELEKTIV DESTRUKTION OF RELATIVELY THE WEAKER MEMBRANE OF THE CANCERCELLS, WHICH HAS RELATIVELI WEAKER CELLMEMBRAN THAN HEALTHY CELLS.

2. USING OF PHOTOHYDRAULIKAL MEKHANIK CHOCK FOR DESTRUKTION OF BACTERIA AND VIRUSES.

3. USING OF PHOTOHYDRAULIKAL MECHANIK CHOCK FOR DESTRUKTION OF STONS AND SAND.
